# EUROPEAN PATENT SPECIFICATION

(11) **EP 0 568 348 B1**
(45) Date of publication and mention of the grant of the patent: **16.07.1997**
(21) Application number: 93303328.4
(22) Date of filing: 28.04.1993
(51) Int. Cl.: C09K 5/04, C10M 105/32

(54) **Liquid compositions containing carboxylic esters**
Flüssige Zusammensetzungen enthaltend Karboxylsäureester
Compositions liquides contenant des esters carboxyliques

(30) Priority: 28.04.1992 US 875911
(43) Date of publication of application: 03.11.1993
(73) Proprietor: The Lubrizol Corporation, Wickliffe, Ohio 44092 (US)
(72) Inventor: Jolley, Scott Ted, Mentor, Ohio 44060 (US); Davis, Kirk Emerson, Chester Township, Ohio 44026 (US)
(74) Representative: Mallalieu, Catherine Louise

(56) References cited:
- EP-A- 0 415 778
- EP-A- 0 435 253
- EP-A- 0 440 069

## Description

This invention relates to liquid compositions comprising at least one fluorine-containing hydrocarbon, and at least one lubricant. More particularly, the invention relates to liquid compositions useful as refrigeration liquids.

Chlorofluorocarbons, generally referred to in the industry as CFCs, have been widely used as propellants in aerosols, although use in aerosols has been diminishing in recent years because of demands of environmentalists for the reduction if not a complete ban on the use of CFCs because of the detrimental effect of CFCs on the stratosphere's ozone layer. CFCs also have been used because of their unique combination of properties as refrigerants, foam-blowing agents, and specialty solvents within the electronics and aerospace industries. Examples of CFCs which have been utilized for these purposes include CFC-13 which is chlorotrifluoromethane, CFC-12 which is dichlorodifluoromethane, and CFC-113 which is 1,2,2-trifluoro-1,1,2-trichloroethane.

Since 1976, when the aerosol industry began to feel the pressure to reduce if not eliminate the use of CFCs, the aerosol industry has progressively moved toward the substitution of hydrocarbon propellants for CFC propellants. The hydrocarbons, such as butane, are readily available and inexpensive, and the quality of the final product generally has been unaffected by the substitution of propellants. However, the problem of finding a safe replacement of CFC refrigerants and foam-blowing agents has been more difficult to solve. Several replacement candidates have been suggested as alternatives to the fully halogenated hydrocarbons, and these include halogenated hydrocarbons containing at least some hydrogen atoms such as HCFC-22 which is difluorochloromethane, HCFC-123 which is 1,1-dichloro-2,2,2-trifluoroethane, HFC-134a which is 1,1,1,2-tetrafluoroethane and HCFC-141b which is 1,1-dichloro-1-fluoroethane.

The ozone depletion potential of these proposed substitutes is significantly less than the ozone depletion potential of the previously used CFCs. The ozone depletion potential is a relative measure of the capability of the material to destroy the ozone layer in the atmosphere. It is a combination of the percentage by weight of chlorine (the atom that attacks the ozone molecule) and the lifetime in the atmosphere. HCFC-22 and HFC-134a generally are recommended as being candidates in refrigerant applications, and HFC-134a is particularly attractive because its ozone depletion potential has been reported as being zero.

In order for any of the replacement materials to be useful as refrigerants, the materials must be compatible with the lubricant utilized in the compressor. The presently used refrigerants such as CFC-12 are readily compatible with mineral lubricating oils which are utilized as the lubricant in air-conditioner compressors. The above-described refrigerant candidates, however, have different solubility characteristics than the refrigerants presently in use. For example, mineral lubricating oil is incompatible (i.e., insoluble) with HFC-134a. Such incompatibility results in unacceptable compressor life in compression-type refrigeration equipment including refrigerators and air-conditioners including auto, home and industrial air-conditioners.

In order to perform as a satisfactory refrigeration liquid, the mixture of refrigerant and lubricant must be compatible and stable over a wide temperature range such as from about 0°C and above 80°C. For some uses, it is generally desirable for the lubricants to be soluble in the refrigerant at concentrations corresponding to the ratios customary in the environment of use, e.g. about 5 to 15%, over a temperature range from -30°, or preferably -40°C, or below, to 80°C or above. In addition to thermal stability, the refrigeration liquids must have acceptable viscosity characteristics which are retained even at high temperatures, and the refrigeration liquid should not have a detrimental effect on materials used as seals in the compressors.

Compositions comprising a tetrafluoroethane and polyoxyalkylene glycols are discussed in U.S. Patent 4,755,316. The compositions are useful in refrigeration systems. Refrigeration oils are described in U.S. Patents 4,248,726 and 4,267,064 which comprise mixtures of a polyglycol and 0.1 to 10% of glycidyl ether type epoxy compounds, or epoxidized fatty acid monoesters, and optionally, epoxidized vegetable oil. The lubricating oils are reported to be useful in refrigerators using a halogen-containing refrigerant such as Freons 11, 12, 13, 22, 113, 114, 500 and 502 (available from DuPont), and in particular with Freon 12 or 22.

U.S. Patent 4,431,557 describes fluid compositions comprised of a fluoro- and chloro-containing refrigerant, a hydrocarbon oil, and an alkylene oxide additive compound which improves the thermal resistance of the oil in the presence of the refrigerant. Examples of hydrocarbon oils include mineral oil, alkyl benzene oil, dibasic acid ester oil, polyglycols, etc. The composition may contain other additives including load-carrying additives such as phosphorus acid esters, phosphoric acid esters, etc. Examples of fluorocarbon refrigerants include R-11, R-12, R-113, R-114, R-500, etc.

U.S. Patent 4,428,854 describes absorption refrigerant compositions for use in refrigeration systems comprising 1,1,1,2-tetrafluoroethane and an organic solvent capable of dissolving the ethane. Among the solvents disclosed are organic amides, acetonitrile, N-methyl pyrroles, N-methyl pyrrolidine, N-methyl-2-pyrrolidone, nitromethane, various dioxane derivatives, glycol ethers, butyl formate, butyl acetate, diethyl oxalate, diethyl malonate, acetone, methyl ethyl ketone, other ketones and aldehydes, triethyl phosphoric triamide, triethylene phosphate, triethyl phosphate, etc.

Stabilized absorption compositions comprising (a) a halogenated hydrocarbon refrigerant, (b) a liquid absorbent of a polyethylene glycol methyl ether, and (c) at least one stabilizer are described in U.S. Patent 4,454,052. Examples of stabilizers include phosphate esters, epoxy compounds, and organotin compounds. The polyethylene glycol methyl ether-type compounds are of the general formula

CH₃-O-(CH₂H₄O)ₙR

wherein n is an integer of 1 to 6, and R is H, CH₃- or CH₃CO-. A variety of halogenated hydrocarbons are described including 1,1-difluoromethane, 1,1,1,2-tetrafluoroethane, etc.

U.S. Patent 4,559,154 relates to absorption heat pumps utilizing as working fluid, a saturated fluorohydrocarbon or fluorohydrocarbon ether having from 3 to 5 carbon atoms. Solvents reported to be useful with such fluorohydrocarbons include ethers such as tetraglyme, amides which can be lactams such as the N-alkyl pyrrolidones, sulfonamides and ureas including cyclic ureas.

According to one aspect of the present invention there is provided a liquid composition (A) at least one fluorine-containing hydrocarbon containing one to three carbon atoms; and a lubricant comprising (B) at least one carboxylic ester of (I) a polyhydroxy compound, and (II) a combination of (a) a nonocarboxylic acylating agent having four or five carbon atoms, (b) a monocarboxylic acylating agent having from 7 to 15 carbon atoms and (c) a polycarboxylic acylating agent.

Included within the scope of the present invention are liquid compositions comprising:
(A) at least one fluorine-containing hydrocarbon containing 1 to 3 carbon atoms and wherein fluorine is the only halogen present, and a lubricant comprising (B) at least one carboxylic ester of (I) dipentaerythritol or tripentaerythritol, and (II) a combination of (a) a monocarboxylic acylating agent having four or five carbon atoms, (b) a branched monocarboxylic acylating agent having from 7 to 15 carbon atoms and (c) a polycarboxylic acylating agent.

Also included within the scope of the present invention are liquid compositions comprising:
(A) at least one fluorine-containing hydrocarbon containing 1 to 3 carbon atoms and wherein fluorine is the only halogen present; and a lubricant comprising (B) at least one carboxylic ester of (I) a trimethylolalkane, and (II) a combination of (a) a monocarboxylic acylating agent having four or five carbon atoms, (b) a branched monocarboxylic acylating agent having from 7 to 15 carbon atoms, and (c) a polycarboxylic acylating agent.

In a further embodiment of the present invention there is provided a liquid composition comprising (A) at least one fluorine-containing hydrocarbon containing 1 to 3 carbon atoms; and a lubricant comprising (B) at least one carboxylic ester of (I) a polyhydroxy compound, and (II) a combination of (a) a monocarboxylic acylating agent having four or five carbon atoms, (b) a monocarboxylic acylating agent having from 7 to 15 carbon atoms, and (c) a polycarboxylic acylating agent; and (C) at least one additive selected from an alkyl phosphite, an alkyl phosphonic acid ester, a nitrogen-containing heterocycle, and a mixture thereof.

The liquid compositions may additionally contain (C) an additive selected from an alkyl phosphite, an alkyl phosphonic acid ester, a nitrogen-containing heterocycle, and a mixture thereof. Liquid compositions also are described wherein the fluorine-containing hydrocarbons also contain other halogens such as chlorine. Methods of lubricating refrigeration systems are also described.

The liquid compositions are useful particularly as refrigeration liquids in refrigerators and air-conditioners including automotive, home, commercial and industrial air-conditioners.

Various preferred features and embodiments of the present invention will now be described by way of non-limiting example.

Throughout this specification and claims, all parts and percentages are by weight, temperatures are in degrees Celsius, and pressures are at or near atmospheric pressure unless otherwise clearly indicated.

As used in this specification and in the appended claims, the terms "hydrocarbyl" and "hydrocarbylene" denote a group having a carbon atom directly attached to the polar group and having a hydrocarbon or predominantly hydrocarbon character within the context of this invention. Such groups include the following:
(1) Hydrocarbon groups; that is, aliphatic, (e.g., alkyl or alkenyl), alicyclic (e.g., cycloalkyl or cycloalkenyl), and the like, as well as cyclic groups wherein the ring is completed through another portion of the molecule (that is, any two indicated substituents may together form an alicyclic group). Such groups are known to those skilled in the art. Examples include methyl, ethyl, octyl, decyl, octadecyl, cyclohexyl, etc.
(2) Substituted hydrocarbon groups; that is, groups containing non-hydrocarbon substituents which, in the context of this invention, do not alter the predominantly hydrocarbon character of the group. Those skilled in the art will be aware of suitable substituents. Examples include halo, hydroxy, alkoxy, etc.
(3) Hetero groups; that is, groups which, while predominantly hydrocarbon in character within the context of this invention, contain atoms other than carbon in a chain or ring otherwise composed of carbon atoms. Suitable hetero atoms will be apparent to those skilled in the art and include, for example, nitrogen, oxygen and sulfur.

In general, no more than about three substituents or hetero atoms, and preferably no more than one, will be present for each 10 carbon atoms in the hydrocarbyl group.

Terms such as "alkyl", "alkylene", etc. have meanings analogous to the above with respect to hydrocarbyl and hydrocarbylene.

The term "hydrocarbon-based" also has the same meaning and can be used interchangeably with the term hydrocarbyl when referring to molecular groups having a carbon atom attached directly to the polar group.

The term "lower" as used herein in conjunction with terms such as hydrocarbyl, hydrocarbylene, alkylene, alkyl, alkenyl, alkoxy, and the like, is intended to describe such groups which contain a total of up to 7 carbon atoms, per se, and includes methyl, ethyl, propyl, butyl, pentyl, hexyl and heptyl groups.

Viscosity, unless otherwise indicated, is kinematic viscosity and is measured by ASTM D-2270.

For purpose of this invention, equivalent weight of polyol is determined by dividing the formula weight of the polyol by the number of hydroxyl groups. Equivalents of polyol is determined by dividing the amount of polyol by its equivalent weight. For polycarboxylic acylating agents or anhydrides, the equivalent weight is determined by dividing the formula weight of the acylating agent or anhydride by the number of carboxylic groups which form esters. For example, an anhydride contributes two carboxyl groups which can form ester. Therefore, the equivalent weight of anhydride, such as succinic anhydride, would be the formula weight of the anhydride divided by the number of carboxyl group. For succinic anhydride, the number is two.

When a compound or component is indicated herein as being "soluble", the compound or component is soluble in the liquid compositions of the invention comprising the fluorine-containing hydrocarbon and the lubricant. For example, a compound or component is considered "soluble" so long as it is soluble in the liquid compositions, even though it may be insoluble in the fluorine-containing hydrocarbon per se.

The term "consisting essentially of" refers to compositions that include the ingredients listed in the claim as well as other ingredients that do not materially affect the basic and novel characteristics of the liquid compositions.

Generally the amount of fluorine-containing hydrocarbon is a major amount for automotive and stationary refrigeration systems. Of course, lower amounts, e.g. less than 50%, of the fluorine-containing hydrocarbon are useful, such as in household refrigerators.

### (A) Fluorine-Containing Hydrocarbon.

The liquid compositions of the present invention comprise, in one instance, a major amount of at least one fluorine-containing hydrocarbon. That is, the fluorine-containing hydrocarbons contain at least one C-H bond as well as C-F bonds. In addition to these two essential types of bonds, the hydrocarbon also may contain other carbon-halogen bonds such as C-Cl bonds. Because the liquid compositions of the present invention are primarily intended for use as refrigerants, the fluorine-containing hydrocarbon preferably contains one to three, or to two carbon atoms, and more preferably two carbon atoms.

As noted above, the fluorine-containing hydrocarbons useful in the liquid compositions of the present invention may contain other halogens such as chlorine. However, in one preferred embodiment, the hydrocarbon contains only carbon, hydrogen and fluorine. These compounds containing only carbon, hydrogen and fluorine are referred to herein as fluorohydrocarbons or hydrofluorocarbons. The hydrocarbons containing chlorine as well as fluorine and hydrogen are referred to as chlorofluorohydrocarbons or hydrochlorofluorocarbons. The fluorine-containing hydrocarbons useful in the composition of the present invention are to be distinguished from the fully halogenated hydrocarbons which have been and are being used as propellants, refrigerants and blowing agents such as CFC-11, CFC-12 and CFC-113 which have been described above.

Specific examples of the fluorine-containing hydrocarbons useful in the liquid compositions of the present invention, and their reported ozone depletion potentials are shown in the following Table I.

**TABLE I**

| Compound Designation | Formula | ODP* |
|---|---|---|
| HCFC-22 | CHClF₂ | 0.05 |
| HCFC-123 | CHCl₂CF₃ | < 0.05 |
| HCFC-141b | CH₃CCl₂F | < 0.05 |
| HFC-134a | CH₂FCF₃ | 0 |

| | | |
|---|---|---|
| * Ozone depletion potential as reported in Process Engineering, pp. 33-34, July, 1988. | | |

Examples of other fluorine-containing hydrocarbons which may be useful in the liquid compositions of the present invention include trifluoromethane (HFC-23), 1,1,1-trifluoroethane (HFC-143a), 1,1-difluoroethane (HFC-152a), 2-chloro-1,1,1,2-tetrafluoroethane (HCFC-124), 1-chloro-1,1,2,2-tetrafluoroethane (HCFC-124a), 1-chloro-1,1-difluoroethane (HCFC-142b), and 1,1,2,2-tetrafluoroethane (HFC-134). Other refrigerants such as perfluoropropane (HFC-218), perfluorocyclopropane (HFC-216), perfluoropropylene oxide, 1,3-perfluoro propylene oxide and pentafluorodimethyl ether may be used with the lubricant. In the refrigerant art, the fluorohydrocarbons are often identified merely with the prefix "R" in place of the above letters. For example HFC-23 is R-23, HCFC-124 is R-124, etc.

In general, fluorine-containing hydrocarbons which are useful as refrigerants are fluoromethanes and fluoroethanes boiling at a relatively low temperature at atmospheric pressure, e.g., below 30°C. Mixtures of fluorine-containing hydrocarbons may be used, and the amount of each fluorohydrocarbon in the mixture may be varied as desired. Examples of fluorohydrocarbon mixtures useful as (A) include: 142(b)/22; 134(a)/23; 22/124/152(a), etc. The useful fluorocarbon refrigerants serve to transfer heat in a refrigeration system by evaporating and absorbing heat at a low temperature and pressure, e.g., near ambient temperature and atmospheric pressure, and by releasing heat on condensing at a higher temperature and pressure.

The amount of fluorine-containing hydrocarbon is the level typically used for the refrigeration system. The liquid compositions of the present invention contain from about 10%, or about 20% up to about 90%, or to about 85% of the fluorine-containing hydrocarbon. In one embodiment, the fluorine-containing hydrocarbon is present in an amount from about 45%, or about 50%, or about 55 % up to about 90%, or to about 80%, or to about 75% by weight of the liquid composition. More generally, the liquid compositions will comprise from about 50% to about 99% by weight of the fluorine-containing hydrocarbon. In another embodiment, the liquid compositions contain from about 70% to about 99% by weight of the fluorine-containing hydrocarbon. When the fluorine-containing hydrocarbon is used at levels greater than 50% by weight of the liquid composition, then the liquid compositions are generally suited for use as automotive and commercial and industrial refrigeration systems.

In one embodiment, the fluorine-containing hydrocarbon is present in an amount from about 10%, or about 25%, or about 30% up to about 55%, or to about 50%, or to about 45% by weight of the lubricant. When the fluorine-containing hydrocarbon is present in an amount less than about 45%, then the liquid compositions are generally suited for household refrigeration systems.

### (B) Carboxylic Esters

In addition to the fluorine-containing hydrocarbons described above, the liquid compositions also contain at least one carboxylic ester of (I) a polyhydroxy compound and (II) a combination of (a) a monocarboxylic acylating agent having four or five carbon atoms, (b) a monocarboxylic acylating agent having from about 7 to about 15 carbon atoms and (c) a polycarboxylic acylating agent.

The carboxylic ester lubricants utilized as component (B) in the liquid compositions are reaction products of the combination of carboxylic acylating agents with polyhydroxy compounds containing at least two hydroxyl groups. The polyhydroxy compounds may be represented by the general formula

R(OH)ₙ (II)

wherein R is a hydrocarbyl group and n is at least 2. The hydrocarbyl group may contain from 4 to about 20 or more carbon atoms, and the hydrocarbyl group may also contain one or more nitrogen and/or oxygen atoms. The polyhydroxy compounds generally will contain from about 2 to about 10 hydroxyl groups and more preferably from about 3 to about 10 hydroxyl groups.

The polyhydroxy compound may contain one or more oxyalkylene groups, and thus, the polyhydroxy compounds include compounds such as polyetherpolyols. The number of carbon atoms and number of hydroxyl groups contained in the polyhydroxy compound used to form the carboxylic esters may vary over a wide range, and it is only necessary the carboxylic ester produced with the polyhydroxy compounds be soluble in the liquid compositions of the present invention.

The polyhydroxy compounds used in the preparation of the carboxylic esters (B) also may contain one or more nitrogen atoms. For example, the polyhydroxy compound may be an alkanolamine containing from 3 to 6 hydroxyl groups. In one preferred embodiment, the polyhydroxy compound is an alkanolamine containing at least two hydroxyl groups and more preferably at least three hydroxyl groups.

The polyhydroxy compounds include trimethylolalkanes, such as trimethylolmethane, trimethylolethane, trimethylolpropane, and trimethylolbutane. The polyhydroxy compound also include pentaerythritol, dipentaerythritol, and tripentaerythritol. In one embodiment, the polyhydroxy compounds are neo polyhydroxy compounds. Specific examples of polyhydroxy compounds useful in the present invention include ethylene glycol, diethylene glycol, triethylene glycol, propylene glycol, dipropylene glycol, glycerol, neopentyl glycol, 1,2-, 1,3- and 1,4-butanediols, pentaerythritol, dipentaerythritol, tripentaerythritol, triglycerol, trimethylolpropane, di-trimethylolpropane, sorbitol, hexaglycerol, 2,2,4-trimethyl-1,3-pentanediol, etc. Preferably, the Mixtures of any of the above polyhydroxy compounds can be utilized.

The above polyhydroxy compounds are reacted with carboxylic acylating agents to form the esters (B). The carboxylic acylating agents include (II) a combination of (a) a monocarboxylic acylating agent having four or five carbon atoms, (b) a monocarboxylic acylating agent having from about 7, or about 8 up to about 15, or to about 12, or to about 10 carbon atoms and (c) a polycarboxylic acylating agent. The acylating agents include acids, anhydrides and lower alkyl esters. In one embodiment, the monocarboxylic acylating agent (IIb) contains 8 or 9 carbon atoms. In one embodiment, the acylating agents are branched.

Examples of carboxylic acylating agents containing a straight chain lower hydrocarbyl group include butyric acid, valeric acid, heptanoic acid, and anhydrides of these acids. Examples of carboxylic acylating agents wherein the hydrocarbyl group is a branched chain hydrocarbyl group include isobutyric acid, 2-ethyl-n-butyric acid, 2-methylbutyric acid, 2,2,4-trimethylpentanoic acid, 2-methylhexanoic acid, 3,5,5-trimethylhexanoic acid, 2-ethylhexanoic acid, isooctanoic acid, isononanoic acid, isoheptanoic acid, isodecanoic acid, neoheptanoic acid, neodecanoic acid, and ISO Acids and NEO Acids available from Exxon Chemical Company, Houston, Texas USA. ISO Acids are isomer mixtures of branched acids and include commercial mixtures such as ISO Heptanoic Acid, ISO Octanoic Acid, and ISO Nonanoic Acid, as well as developmental products such as ISO Decanoic Acids and ISO 810 Acid. Of the ISO Acids, ISO Octanoic acid and ISO Nonanoic acid are preferred. In one embodiment, the ISO acid may be isopentanoic acid (available commercially from Union Carbide), which is a mixture of 66% by weight valeric acid and 34% by weight 2-methylbutyric acid. Neo acids include commercially available mixtures such as NEO Pentanoic Acid, NEO Heptanoic Acid, and NEO Decanoic Acid, as well as developmental products such as ECR-909 (NEO C₉) Acid, and ECR-903 (NEO C₁₂₁₄) Acid and commercial mixtures of branched chain carboxylic acids such as the mixture identified as NEO 1214 acid from Exxon.

The polycarboxylic acylating agents may contain 2 up to about 4, or to about 3, preferably two carboxylic groups. In one embodiment, the polycarboxylic acylating agent is a dicarboxylic acylating agent. Examples of useful polycarboxylic acylating agents include maleic acid or anhydride, succinic acid or anhydride, adipic acid or anhydride, oxalic acid or anhydride, pimelic acid or anhydride, glutaric acid or anhydride, suberic acid or anhydride, azelaic acid or anhydride, sebacic acid or anhydride, etc.

The carboxylic ester (B) may conveniently include compounds having the general formula

R[OC(O)R¹]ₙ (I)

wherein R is a hydrocarbyl group, each R¹ is independently hydrogen, a straight chain lower hydrocarbyl group, a branched chain hydrocarbyl group, or a straight chain hydrocarbyl group containing from 8 to about 22 carbon atoms provided that at least one R¹ group is hydrogen, a lower straight chain hydrocarbyl or a branched chain hydrocarbyl group, or a carboxylic acid- or carboxylic acid ester-containing hydrocarbyl group, and n is at least 2.

Carboxylic acids that may also be utilized in the preparation of the carboxylic esters useful in the liquid compositions include those of the following general formula

R¹COOH (III)

wherein R¹ is (a) H, (b) a straight or branch chain lower hydrocarbyl group (preferably about three to five, or to four carbon atoms), (c) a branched chain hydrocarbyl group, or (d) a mixture of one or both of (b) and (c) with a straight chain hydrocarbyl group containing from about 8 to about 22 carbon atoms or (e) a carboxylic acid- or carboxylic acid ester-containing hydrocarbyl group. Stated otherwise, at least one R¹ group in the ester product of Formula I must contain a lower straight chain hydrocarbyl group or a branched chain hydrocarbyl group. The straight chain lower hydrocarbyl group (R¹) contains from 1 to about 7 carbon atoms, and in a preferred embodiment, contains from 1 to about 5 carbon atoms. The branched chain hydrocarbyl group may contain any number of carbon atoms and will generally contain from 4 to about 20 carbon atoms. In one preferred embodiment, the branched chain hydrocarbon group contains from 5 to 20 carbon atoms and in a more preferred embodiment, contains from about 5 to about 14 carbon atoms. The higher molecular weight straight chain hydrocarbyl group containing from 8 to about 22 carbon atoms will contain in some embodiments, from 8 to about 18 carbon atoms, and in more preferred embodiments from 8 to about 14 carbon atoms.

In one embodiment, the branched chain hydrocarbyl groups are characterized by the structure

-C(R²)(R³)(R⁴)

wherein R², R³ and R⁴ are each independently alkyl groups, and at least one of the alkyl groups contains two or more carbon atoms. Such branched chain alkyl groups, when attached to a carboxyl group are referred to in the industry as neo groups and the acids are referred to a neo acid. The neo acids are characterized as having alpha-alpha-, disubstituted hydrocarbyl groups. In one embodiment, R² and R³ are methyl groups and R⁴ is an alkyl group containing two or more carbon atoms.

Any of the above hydrocarbyl groups (R¹) may contain one or more carboxy groups or carboxy ester groups such as -COOR⁵ wherein R⁵ is a lower alkyl, hydroxyalkyl or a hydroxyalkyloxy group. Such substituted hydrocarbyl groups are present, for example, when the carboxylic acid R¹COOH (III) is a dicarboxylic acid or a monoester of a dicarboxylic acid. Generally, however, the acid R¹COOH (III) is a monocarboxylic acid since polycarboxylic acids tend to form polymeric products if the reaction conditions and amounts of reactants are not carefully regulated. Mixtures of monocarboxylic acids and minor amounts of dicarboxylic acids or anhydrides are useful in preparing the esters (I).

The ester is prepared from one of the polyhydroxy compounds described above with a combination of acylating agents including a monocarboxylic acylating agent having from about 7, or about 8 up to about 15, or to about 12, or to about 10 carbon atoms. In one embodiment, the monocarboxylic acylating agent may be linear or branched. Particularly useful monocarboxylic acylating agents include branched monocarboxylic acylating agents containing 8 or 9 carbon atoms.

Carboxylic acylating agents which can be utilized in the preparation of the carboxylic esters include the acylating agents containing a straight chain hydrocarbyl group containing from 8 to about 22 carbon atoms. These higher molecular weight straight chain acylating agents can be utilized only in combination with the other acylating agents described above since the higher molecular weight straight chain acylating agents are not soluble in the fluorohydrocarbons. Examples of such higher molecular weight straight chain acylating agents include decanoic acid, dodecanoic acid, stearic acid, lauric acid, behenic acid, etc.

In another embodiment, the carboxylic acylating agents utilized to prepare the carboxylic esters may comprise a mixture of a major amount of monocarboxylic acylating agents and a minor amount of dicarboxylic acylating agents. The presence of the dicarboxylic acylating agents results in the formation of esters of higher viscosity. The complex esters are formed by having a substantial portion of the dicarboxylic acids or anhydrides react with more than one polyol. The reaction is generally coupling of polyols through the dicarboxylic acid or anhydride. Examples of mixtures of mono- and dicarboxylic acylating agents include succinic anhydride and 3,5,5-trimethylhexanoic acid; azelaic acid and 2,2,4-trimethylpentanoic acid; adipic acid and 3,5,5-trimethylhexanoic acid; sebacic acid and isobutyric acid; adipic and a mixture of 50 parts 3,5,5-trimethylhexanoic acid and 50 parts neoheptanoic acid; and neoheptanoic acid and a mixture of 50 parts adipic acid and 50 parts sebacic acid. The use of mixtures containing larger amounts of dicarboxylic acylating agents should be avoided since the product ester will contain larger amounts of polymeric esters, and such mixtures may be insoluble in the fluorohydrocarbons. An example of such a mixture is 80 parts of neoheptanoic acid and 20 parts of succinic acid. Viscosity and average molecular weight of the ester can be increased by increasing the amount of dicarboxylic acylating agent and decreasing the amount of monocarboxylic acylating agent.

The carboxylic esters of the liquid compositions are prepared, as mentioned above, by reacting the combination of carboxylic acids or anhydrides with at least one polyhydroxy compound containing at least two hydroxyl groups. The formation of esters by the interaction of carboxylic acids and alcohols is acid catalyzed and is a reversible process which can be made to proceed to completion by use of a large amount of alcohol or carboxylic acylating agent, or by removal of the water as it is formed in the reaction. If the ester is formed by transesterification of a lower molecular weight carboxylic ester, the reaction can be forced to completion by removal of water, or the low molecular weight alcohol or acid formed as a result of a transesterification reaction. The esterification reaction can be catalyzed by either organic acids or inorganic acids. Examples of inorganic acids include sulfuric acids and acidified clays. A variety of organic acids can be utilized including paratoluenesulfonic acid, and acidic resins, such as Amberlyst 15, etc. Organometallic catalysts include, for example, tetraisopropoxy orthotitanate.

The amounts of carboxylic acylating agents and polyhydroxy compounds included in the reaction mixture may be varied depending on the results desired. If it is desired to esterify all of the hydroxyl groups containing in the polyhydroxy compounds, sufficient carboxylic acylating agent should be included in the mixture to react with all of the hydroxyl groups. When mixtures of the alcohols are reacted with a polyhydroxy compound, the carboxylic acylating agents can be reacted sequentially with the polyhydroxy compounds or a mixture of carboxylic acylating agents can be prepared and the mixture reacted with the polyhydroxy compounds. In one preferred embodiment, the polyhydroxy compound is first reacted with one carboxylic acid, generally, the higher molecular weight branched chain or straight chain carboxylic acylating agent followed by reaction with a straight chain lower hydrocarbyl carboxylic acid.

Throughout the specification and claims, it should be understood that the esters also can be formed by reaction of the polyhydroxy compound with the anhydrides of any of the above-described carboxylic acids. For example, esters are easily prepared by reacting the polyhydroxy compounds either with acetic acid or acetic anhydride.

The amount of the carboxylic acylating agents (a), (b), and (c) may be varied to obtain a product for the desired result. In preparing the esters from (II) a combination of (a) monocarboxylic acylating agents having four or five carbon atoms, (b) monocarboxylic acylating agents having from about 7 to about 15 carbon atoms, and (c) polycarboxylic acylating agents, one equivalent of polyhydroxy compound is reacted with from about 0.1, or about 0.2 up to about 0.83, or to about 0.8, or to about 0.7 moles of (a); from about 0.1, or about 0.2 up to about 0.83, or to about 0.8, or to about 0.7 moles of (b); and from about 0.07, or about 0.1 up to about 0.3, or to about 0.2 moles of (c). In one embodiment, each equivalent of polyhydroxy compound is reacted with from about 0.5, or about 0.55, or to about 0.6 up to about 0.8, or to about 0.7, or to about 0.65 mole of (a); from about 0.15, or about 0.2 up to about 0.3, or to about 0.25 moles of (b); and from about 0.075, or about 0.1 up to about 0.2, or to about 0.15 moles of (c). The molar sum of (a), (b) and (c) will generally equal about one for each equivalent of polyhydroxy compound; of course, more than one equivalent of acylating agent, and particularly of monocarboxylic acid, may be used.

In one embodiment, the esters are made by reacting a polyol with a mixture of a dicarboxylic acylating agent and monocarboxylic acylating agents. Preferably, one equivalent of polyol is reacted with from about 0.07, preferably from about 0.17 to about 0.33, preferably to about 0.23 moles of dicarboxylic acylating agent and from about 0.67, preferably from about 0.77 to about 0.93, preferably to about 0.83 moles of monocarboxylic acylating agent.

The formation of esters by the reaction of carboxylic acylating agents with the polyhydroxy compounds described above can be effected by heating the acylating agents, the polyhydroxy compounds, with or without an acid catalyst to an elevated temperature while removing water, or low molecular weight alcohols or acids formed in the reaction. Generally, temperatures of from about 75°C to about 200°C or higher are sufficient for the reaction. The reaction is completed when water, or low molecular weight alcohol or acid is no longer formed, and such completion is indicated when water, or low molecular weight alcohols or acids can no longer be removed by distillation.

In some instances, it is desired to prepare carboxylic esters wherein not all of the hydroxyl groups have been esterified. Such partial esters can be prepared by the techniques described above and by utilizing amounts of the acid or acids which are insufficient to esterify all of the hydroxyl groups.

The following examples illustrate the preparation of various carboxylic esters which are useful as lubricants (B) in the liquid compositions of the invention.

### Example 1

Esters are prepared by reacting mixtures of isononanoic acid (1) isooctanoic acid (2), isobutyric acid (3), adipic acid (4) and pentaerythritol (5), in the presence of a tetraisopropoxy orthotitanate catalyst. The reactants are charged to a flask and heated until reaction ceases, as indicated by termination of water collection in a distillation trap, at which point the reaction mixture has reached about 220°C. A vacuum is applied to remove volatile components, and the flask contents are cooled and filtered to produce the liquid ester product.

Properties of the products are as follows:

| | Moles | | | | | Catalyst |
|---|---|---|---|---|---|---|
| Product | (1) | (2) | (3) | (4) | (5) | grams |
| A | 7 | 7 | 7 | 1.5 | 6 | 5 |
| B | 7.2 | 7.2 | 6 | 1.8 | 6 | 5 |

| Product | Viscosity, cSt | | Molecular |
|---|---|---|---|
| | 40°C | 100°C | Weight |
| A | 149.5 | 14.0 | 733 |
| B | 194 | 16.9 | 802 |

### Example 2

A vessel reaction is charged with 372 parts (1 mole) of tripentaerythritol, 650 parts (5 moles) of neo heptanoic acid, 204 parts (2 moles) of neo pentanoic acid, 59 parts (0.5 mole) of succinic acid, and 4 parts of tetra-isopropoxy titanate. The mixture is heated to 150°C. and the temperature is maintained for 48 hours. The mixture becomes thick and the temperature is raised to 200°c > and the temperature is maintained for 24 hours. The product is filtered and the residue is the product. The residue has a kinematic viscosity of 830 cSt.

### Example 3

An ester is prepared by the procedure of Example 2 using 134 parts (1 mole) of trimethylolpropane, 269 parts (1.9 moles) of isooctanoic acid, 77 parts (0.75 mole) of Neo pentanoic acid, 34 parts (0.2 mole) of azelaic acid, and 4 parts of tetraisopropoxytitanate.

### Example 4

An ester is prepared by the procedure of Example 2 using 136 parts (1 mole) of pentaerythritol, 325 parts (2.5 moles) of neo heptanoic acid, 88 parts (1 mole) of isobutyric acid, 37 parts (0.25 mole) of adipic acid, and 4 parts of tetraisopropoxytitanate.

### Example 5

An ester is prepared by the procedure of Example 2 using 372 parts (1 mole) of tripentaerythritol, 790 parts (5 moles) of 3,5,5-trimethylhexanoic acid, 204 parts (2 moles) of isopentanoic acid, 73 parts (0.5 mole) of adipic acid, and 4 parts of tetraisopropoxytitanate.

### Example 6

An ester is prepared by the procedure of Example 2 using 372 parts (1 mole) of tripentaerythritol, 260 parts (2 moles) of neo heptanoic acid, 510 parts (5 moles) of neo pentanoic acid, 59 parts (0.5 mole) of succinic acid, and 4 parts of tetraisopropoxytitanate.

### Example 7

An ester is prepared by the procedure of Example 2 using 136 parts (1 mole) of pentaerythritol, 158 parts (1 mole) of 3,5,5-trimethylhexanoic acid, 220 parts (2.5 moles) of isobutyric acid, 37 parts (0.25 mole) of adipic acid, and 4 parts of tetraisopropoxytitanate.

In one embodiment, the carboxylic esters preferably contain branched alkyl groups and generally are free of acetylenic and aromatic unsaturation. Some compounds which contain such unsaturation may be insoluble in the fluorine-containing hydrocarbons. The soluble lubricants of this invention also are preferably free of olefinic unsaturation except that some olefinic unsaturation may be present so long as the lubricant is soluble.

The carboxylic esters (I) are soluble in the fluorine-containing hydrocarbons and, in particular, in the fluorohydrocarbons such as 1,1,1,2-tetrafluoroethane. The lubricants are soluble over a wide temperature range and, in particular, at low temperatures. The solubility of the lubricants in fluorohydrocarbons such as 1,1,1,2-tetrafluoroethane at low temperatures is determined in the following manner. The lubricant (0.5 gram) is placed in a thick-walled glass vessel equipped with a removable pressure gauge. The tetrafluoroethane (4.5 grams) is condensed into the cooled (-40°C) glass vessel, and the contents are warmed to the desired temperature and mixed to determine if the lubricant is soluble in the tetrafluoroethane. If soluble, the temperature of the mixture is reduced until a separation and/or precipitate is observed. The results of this solubility test conducted with an example of the carboxylic ester lubricants are summarized in the following Table II.

**TABLE II**

| Liquid Containing Product of Example | Solubility °C (ppt.) |
|---|---|
| 2 | -30 |

In one embodiment, the liquid compositions comprise a major amount of a fluorine-containing hydrocarbon and a minor amount of at least one soluble organic lubricant comprising at least one carboxylic ester (I). By "major amount" is meant an amount greater than 50% by weight such as 50.5%, 70%, 99%, etc. The term "minor amount" includes amounts less than 50% by weight such as 1%, 5%, 20%, 30% and up to 49.9%. In one embodiment, the liquid composition will comprise from about 70% to about 99% of the fluorine-containing hydrocarbon and from about 1 to about 30% by weight of the lubricant. In other embodiments, the liquid compositions may contain from about 5% to about 20% by weight of the lubricant.

The liquid compositions may additionally contain (C) at least one additive selected from the group consisting of an alkyl phosphite, an alkyl phosphonic acid ester, a nitrogen-containing heterocycle, and a mixture thereof. The phosphite and/or the alkyl phosphonic acid ester are present in an amount sufficient to provide antiwear and/or extreme pressure properties to the lubricant and liquid composition. The phosphite and/or the alkyl phosphonic acid ester are present in an amount to provide 0.001%, or to 0.015%, or about 0.025%, to about 1%, or to about 0.5%, or to about 0.2% by weight phosphorus to the lubricant. The nitrogen-containing heterocycle is present in an amount from about 0.001%, or about 0.02%, or about 0.03% up to about 5%, or to about 2%, or to about 1%, or to about 0.5% by weight of the lubricant.

The phosphite and/or the alkyl phosphonic acid ester provide beneficial antiwear and extreme pressure properties to the liquid compositions. The phosphite may be a dialkyl or trialkyl phosphite, preferably a dialkyl phosphite. The alkyl phosphonic acid ester may be an alkyl phosphonic acid diester, preferably a dialkylester. The alkyl groups of the phosphite and the phosphonic acid ester independently contain from 1, or about 3 to about 20, or to about 18, or to about 8 carbon atoms. In one embodiment, the phosphite and the phosphonic acid ester have alkyl groups independently containing from about 3 to about 6, or to about 5 carbon atoms. A number of dialkyl phosphites are commercially available, such as lower dialkyl phosphites, which are preferred. Lower dialkyl phosphites include dimethyl, diethyl, dipropyl, dibutyl, dipentyl and dihexyl phosphites. Phosphites and their preparation are known and many phosphites are available commercially. Also mixed alkyl phosphites, made from a mixture of alcohols, are useful in the present invention. Examples of mixtures of alcohols include ethyl and butyl alcohol; propyl and pentyl alcohol; and methyl and pentyl alcohol. A particularly useful phosphite is dibutyl phosphite.

Alkyl phosphonic acid esters are prepared by means known to those in the art. For example, alkyl phosphonic acid esters may be prepared by reacting an alkyl halide with a trialkyl phosphite. Examples of alkyl phosphonic acid esters include diethyl, butylphosphonate; dibutyl,butylphosphonate; 2-ethylhexyl,2-ethylhexylphosphonate, etc.

The lubricant may additionally contain a nitrogen-containing heterocycle, such as dimercaptothiadiazoles, triazoles, amino-mercaptothiadiazoles, imidazoles, thiazoles, tetrazoles, hydroxyquinolines, oxazolines, imidazolines, thiophenes, indoles, indazoles, quinolines, benzoxazines, dithiols, oxazoles, oxatriazoles, pyridines, piperazines, triazines, and derivatives of any one or more thereof. In one embodiment, the nitrogen containing heterocycle is a triazole or derivative thereof, a thiazole or derivative thereof, a mercaptothiazole or derivative thereof and a thiadiazole or derivative thereof, preferably a triazole or derivative thereof. These additives provide metal deactivating, metal passivating and corrosion controlling character to the liquid compositions. Examples of useful metal deactivators include dimercaptothiadiazoles and derivatives thereof, substituted and unsubstituted triazoles (e.g., benzotriazole, tolyltriazole, octylbenzotriazole, and the like), mercaptobenzothiazoles, etc. Examples of these compounds are benzotriazole, alkyl-substituted benzotriazole (e.g., tolyltriazole, ethylbenzotriazole, hexylbenzotriazole, octylbenzotriazole, etc.), aryl-substituted benzotriazole (e.g., phenol benzotriazoles, etc.), and alkylaryl- or arylalkyl-substituted benzotriazole and substituted benzotriazoles where the substituent may be hydroxy, alkoxy, halo (especially chloro), nitro, carboxy and carboxyalkoxy. Preferably, the triazole is a benzotriazole or an alkylbenzotriazole in which the alkyl group contains 1 to about 20 carbon atoms, preferably 1 to about 8 carbon atoms.

The nitrogen containing heterocycle may also be the reaction product of at least one of the above benzotriazoles, at least one amine and an aldehyde or aldehyde precursor. The amine can be one or more mono or polyamines. These monoamines and polyamines can be primary amines, secondary amines or tertiary amines. The amines may be monoamines or polyamines. Examples of polyamines include polyalkylenepolyamines, and heterocyclic polyamines. The polyalkyleneamines include polyethylenepolyamines, such as diethylenetriamine, triethylenetrimine, tetraethylenepentaamine, etc.

The aldehyde is typically a hydrocarbon-based aldehyde, preferably a lower aliphatic aldehyde. Suitable aldehydes include formaldehyde, benzaldehyde, acetalde-hyde, the butyraldehydes, hydroxybutyraldehydes and heptanals, as well as aldehyde precursors which react as aldehydes under the conditions of the reaction such as paraformaldehyde, paraldehyde, formalin and methanal. Formaldehyde and its precursors (e.g., paraformaldehyde, trioxane) are preferred. Mixtures of aldehydes may be used.

An example of a useful triazole derivative is Reomet® 39. This material is a triazole derivative available commercially from Ciba-Geigy corp.

The liquid compositions of the present invention are characterized as having improved thermal and chemical stability over a wide temperature range. The liquid compositions have improved antiwear and corrosion stability properties. The liquid compositions have beneficial viscosity properties. Preferably the liquid compositions have a viscosity of 5-400 centistokes (cSt) measured at 40°C.

Liquid compositions containing carboxylic esters derived from polyols such as neopentylglycol, trimethylol propane and pentaerythritol, have beneficial thermal and hydrolytic stability. Liquid compositions containing carboxylic esters derived from branched acids, such as iso or neo acids, preferably neo acid, have improved thermal and hydrolytic stability. In one embodiment, the carboxylic esters are derived from the above polyols, a polycarboxylic acid and an iso or neo acid. The liquid composition may contain one carboxylic ester reaction product or in another embodiment, the liquid compositions may contain a blend of two or more carboxylic ester reaction products. A liquid composition of a desired viscosity may be prepared by blending a higher viscosity carboxylic ester with a lower viscosity carboxylic ester. Other additives, if soluble in the liquid, known to be useful for improving the properties of halogen-containing hydrocarbon refrigerants can be included in the liquid compositions of the present invention to improve the characteristics of the liquid as a refrigerant. However, hydrocarbon oils such as mineral oil generally are not included in and are most often excluded from the liquid compositions of the invention, particularly when the fluorine-containing hydrocarbon contains no other halogens. Hydrocarbon lubricants, however, may be present if the liquid compositions are used to retrofit a compressor system which had previously used a hydrocarbon lubricant.

Other additives may be included in the liquid compositions of the present invention to enhance the performance of the liquids include extreme-pressure and anti-wear agents, oxidation and thermal-stability improvers, corrosion-inhibitors, viscosity-index improvers, pour point and/or floc point depressants, detergents, dispersants, anti-foaming agents, viscosity adjusters, metal deactivators, etc. As noted above, these supplementary additives must be soluble in the liquid compositions of the invention. Included among the materials which may be used as extreme-pressure and anti-wear agents are phosphates, phosphate esters, thiophosphates such as zinc diorganodithiophosphates, chlorinated waxes, sulfurized fats and olefins, organic lead compounds, fatty acids, molybdenum complexes, borates, halogen-substituted phosphorous compounds, sulfurized Diels Alder adducts, organic sulfides, metal salts of organic acids, etc. Sterically hindered phenols, aromatic amines, dithiophosphates, sulfides and metal salts of dithioacids are useful examples of oxidation and thermal stability improvers. Compounds useful as corrosion-inhibitors include organic acids, organic amines, organic phosphates, organic alcohols, metal sulfonates, etc. VI improvers include polyolefins such as polyesterbutene, polymethacrylate, polyalkyl styrenes, etc. Pour point and floc point depressants include polymethacrylates, ethylene-vinyl acetate copolymers, succinamic acid-olefin copolymers, ethylene-alpha olefin copolymers, etc. Detergents include sulfonates, long-chain alkyl-substituted aromatic sulfonic acids, phosphonates, phenylates, metal salts of alkyl phenols, alkyl phenol-aldehyde condensation products, metal salts of substituted salicylates, etc. Silicone polymers are a well known type of anti-foam agent. Viscosity adjusters are exemplified by polyisobutylene, polymethacrylates, polyalkyl styrenes, naphthenic oils, alkyl benzene oils, polyesters, polyvinyl chloride, polyphosphates, etc.

The following examples (TABLE 1) relate to formulations which are useful as organic lubricant (B).

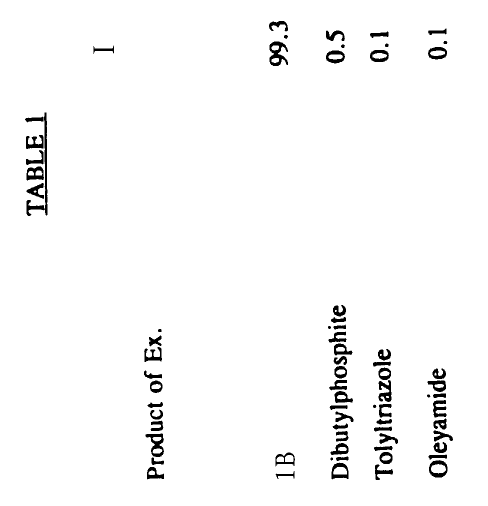

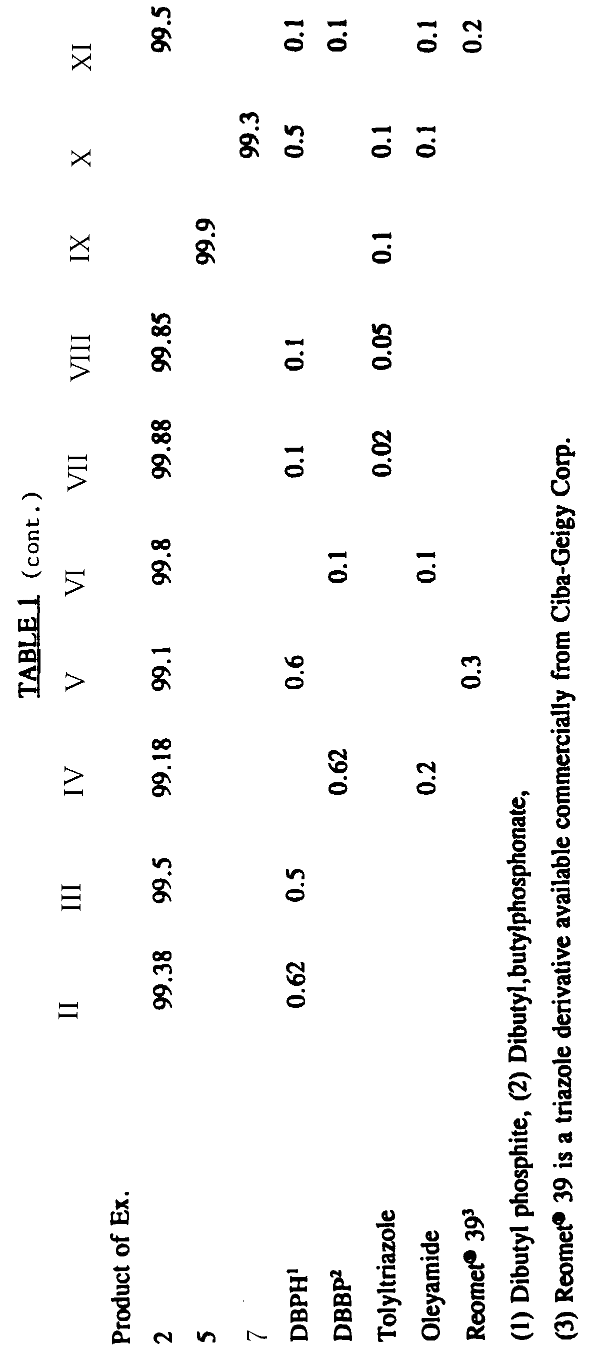

The liquid compositions of the present invention are particularly useful as refrigerants in various refrigeration systems which are compression-type systems such as refrigerators, freezers, and air-conditioners including automotive, home and industrial air-conditioners. The following examples are illustrative of the liquid compositions of the present invention.

| | Parts by Wt. |
|---|---|
| Example A | |
| 1,1,1,2-tetrafluoroethane (HFC-134a) | 90 |
| Lubricant of Example 2 | 10 |
| DBPH | 0.625 |
| Benzotriazole | 0.02 |
| Example B | |
| 1,1,1,2-tetrafluoroethane | 85 |
| Lubricant of Example 2 | 15 |
| DBBP | 0.2 |
| Example C | |
| HFC-134a | 60 |
| Lubricant of Example 2 | 40 |
| DBPH | 0.1 |
| benzotriazole | 0.01 |
| Example D | |
| HFC-134a | 55 |
| Product of Example 5 | 45 |
| tolyltriazole | 0.025 |
| Example E | |
| HFC-134a | 85 |
| Product of Example 6 | 15 |
| benzotriazole | 0.02 |

This application is related to U.S. Serial No. 07/728,441 filed July 11, 1991, which is a continuation-in-part of U.S. Serial No. 608,600 filed on October 30, 1990, which is a continuation of Serial No. 343,087 filed on April 25, 1989 and now abandoned.

## Claims

1. A liquid composition comprising:
(A) at least one fluorine-containing hydrocarbon containing 1 to 3 carbon atoms, and a lubricant comprising (B) at least one carboxylic ester of (I) a polyhydroxy compound and (II) a combination of (a) a monocarboxylic acylating agent having four or five carbon atoms, (b) a monocarboxylic acylating agent having from 7 to 15 carbon atoms and (c) a polycarboxylic acylating agent.

2. A liquid composition of claim 1 wherein fluorine is the only halogen in the fluorine-containing hydrocarbon (A).

3. A liquid composition of either of claims 1 and 2 wherein the fluorine-containing hydrocarbon (A) is 1,1,1,2-tetrafluoroethane.

4. A liquid composition of any preceding claim wherein the polyhydroxy compound (I) is a trimethylolalkane.

5. A liquid composition of any preceding claim wherein the polyhydroxy compound (I) is trimethylolpropane.

6. A liquid composition of any preceding claim wherein the polyhydroxy compound (I) contains an average of five or more hydroxyl groups.

7. A liquid composition of any preceding claim wherein the polyhydroxy compound (I) is dipentaerythritol or tripentaerythritol.

8. A liquid composition of any preceding claim wherein the monocarboxylic acylating agent (IIa) is isobutyric, valeric, 2-methylbutyric, or neopentanoic acid or anhydride.

9. A liquid composition of any preceding claim wherein the monocarboxylic acylating agent (IIb) is branched.

10. A liquid composition of claim 9 wherein the monocarboxylic acylating agent (IIb) is a branched monocarboxylic acylating agent with 8 or 9 carbon atoms.

11. A liquid composition of any preceding claim wherein the polycarboxylic acylating agent (IIc) is a dicarboxylic acylating agent.

12. A liquid composition of any preceding claim wherein the polycarboxylic acylating agent (IIc) is an acylating agent selected from maleic, succinic, adipic, and azelaic acylating agents.

13. A liquid composition of any preceding claim further comprising (C) an additive selected from an alkyl phosphite, an alkyl phosphonic acid ester, a nitrogen containing heterocycle, and a mixture thereof.

14. A liquid composition of claim 13 wherein (C) is an alkyl phosphite or alkyl phosphonic acid ester independently having from 1 to 20 carbon atoms in each alkyl group.

15. A liquid composition of claim 13 wherein (C) is a phosphite or alkyl phosphonic acid ester independently having from 3 to 8 carbon atoms in each alkyl group.

16. A liquid composition of claim 13 wherein (C) is dibutylphosphite.

17. A liquid composition of claim 13 wherein (C) is dibutyl, butyl phosphonate.

18. A liquid composition of claim 13 wherein (C) is a triazole or derivative thereof.

19. A liquid composition of claim 13 wherein (C) is tolyltriazole, benzotriazole, or a reaction product of a triazole, an amine, and an aldehyde or aldehyde precursor.

20. A liquid composition of claim 13 wherein (C) is a mixture of an alkyl phosphite independently having from 1 to 20 carbon atoms in each alkyl group and a triazole or derivative thereof.

21. A method of lubricating a refrigeration system comprising the steps of introducing into the refrigeration system a composition according to any one of claims 1 to 20; and operating the system.

22. A process for the preparation of a liquid composition comprising admixing:
(A) at least one fluorine-containing hydrocarbon containing 1 to 3 carbon atoms, and a lubricant comprising (B) at least one carboxylic ester of (I) a polyhydroxy compound and (II) a combination of (a) a monocarboxylic acylating agent having four or five carbon atoms, (b) a monocarboxylic acylating agent having from 7 to 15 carbon atoms and (c) a polycarboxylic acylating agent.

## Patentansprüche

1. Flüssige Zusammensetzung umfassend :
(A) mindestens einen Fluor-enthaltenden Kohlenwasserstoff mit 1 bis 3 Kohlenstoffatomen und ein Schmiermittel, umfassend (B) mindestens einen Carbonsäureester von (I) einer Polyhydroxyverbindung und (II) eine Kombination von (a) einem Monocarbonsäureacylierungsmittel mit 4 oder 5 Kohlenstoffatomen, (b) ein Monocarbonsäureacylierungsmittel mit 7 bis 15 Kohlenstoffatomen und (c) ein Polycarbonsäureacylierungsmittel.

2. Flüssige Zusammensetzung nach Anspruch 1, in der Fluor das einzige Halogen in dem Fluor-enthaltenden Kohlenwasserstoff (A) ist.

3. Flüssige Zusammensetzung nach einem der Ansprüche 1 oder 2, wobei der Fluorenthaltende Kohlenwasserstoff (A) 1,1,1,2-Tetrafluorethan ist.

4. Flüssige Zusammensetzung nach einem der vorstehenden Ansprüche, wobei die Polyhydroxyverbindung (I) ein Trimethylolalkan ist.

5. Flüssige Zusammensetzung nach einem der vorstehenden Ansprüche, wobei die Polyhydroxyverbindung (I) Trimethylolpropan ist.

6. Flüssige Zusammensetzung nach einem der vorstehenden Ansprüche, wobei die Polyhydroxyverbindung (I) durchschnittlich 5 oder mehr Hydroxylgruppen enthält.

7. Flüssige Zusammensetzung nach einem der vorstehenden Ansprüche, wobei die Polyhydroxyverbindung (I) Dipentaerythrit oder Tripentaerythrit ist.

8. Flüssige Zusammensetzung nach einem der vorstehenden Ansprüche, wobei das Monocarbonsäureacylierungsmittel (IIa) Isobutan-, Valerian-, 2-Methylbutan- oder Neopentansäure oder -säureanhydrid ist.

9. Flüssige Zusammensetzung nach einem der vorstehenden Ansprüche, wobei das Monocarbonsäureacylierungsmittel (IIb) verzweigtkettig ist.

10. Flüssige Zusammensetzung nach Anspruch 9, wobei das Monocarbonsäureacylierungsmittel (IIb) ein verzweigtkettiges Monocarbonsäureacylierungsmittel mit 8 oder 9 Kohlenstoffatomen ist.

11. Flüssige Zusammensetzung nach einem der vorstehenden Ansprüche, wobei das Polycarbonsäureacylierungsmittel (IIc) ein Dicarbonsäureacylierungsmittel ist.

12. Fiüssige Zusammensetzung nach einem der vorstehenden Ansprüche, wobei das Polycarbonsäureacylierungsmittel (IIc) ein Acylierungsmittel ausgewählt aus Maleinsäure-, Bernsteinsäure-, Adipinsäure- und Azelainsäureacylierungsmitteln ist.

13. Flüssige Zusammensetzung nach einem der vorstehenden Ansprüche, des weiteren umfassend (C) ein Additiv ausgewählt aus einem Alkylphosphit, einem Alkylphosphonsäureester, einem Stickstoff-enthaltenden Heterocyclus und einem Gemisch davon.

14. Flüssige Zusammensetzung nach Anspruch 13, wobei (C) ein Alkylphosphit oder Alkylphosphonsäureester mit unabhängig 1 bis 20 Kohlenstoffatomen in jedem Alkylrest ist.

15. Flüssige Zusammensetzung nach Anspruch 13, wobei (C) ein Alkylphosphit oder Alkylphosphonsäureester mit unabhängig 3 bis 8 Kohlenstoffatomen in jedem Alkylrest ist.

16. Flüssige Zusammensetzung nach Anspruch 13, wobei (C) Dibutylphosphit ist.

17. Flüssige Zusammensetzung nach Anspruch 13, wobei (C) Dibutyl- butylphosphonat ist.

18. Flüssige Zusammensetzung nach Anspruch 13, wobei (C) ein Triazol oder ein Derivat davon ist.

19. Flüssige Zusammensetzung nach Anspruch 13, wobei (C) Tolyltriazol, Benzotriazol oder ein Reaktionsprodukt aus einem Triazol, einem Amin und einem Aldehyd oder einer Aldehydvorstufe ist.

20. Flüssige Zusammensetzung nach Anspruch 13, wobei (C) ein Gemisch eines Alkylphosphits mit unabhängig 1 bis 20 Kohlenstoffatomen in jedem Alkylrest und eines Triazols oder einem Derivat davon ist.

21. Verfahren zum Schmieren von Kühlsystemen, umfassend die Schritte des Einführens einer Zusammensetzung gemäß einem der Ansprüche 1 bis 20 in das Kühlsystem und Betreiben des Systems.

22. Verfahren zur Herstellung einer flüssigen Zusammensetzung, umfassend das Vermischen von:
(A) mindestens einem Fluor-enthaltenden Kohlenwasserstoff mit 1 bis 3 Kohlenstoffatomen und einem Schmiermittel, umfassend (B) mindestens einen Carbonsäureester von (I) einer Polyhydroxyverbindung und (II) eine Kombination aus (a) einem Monocarbonsäureacylierungsmittel mit 4 oder 5 Kohlenstoffatomen, (b) einem Monocarbonsäureacylierungsmittel mit 7 bis 15 Kohlenstoffatomen und (c) einem Polycarbonsäureacylierungsmittel.

## Revendications

1. Une composition liquide comprenant :
(A) au moins un hydrocarbure fluoré contenant 1 à 3 atomes de carbone, et un lubrifiant comprenant (B) au moins un ester carboxylique de (I) un composé polyhydroxylé et de (II) une combinaison de (a) un agent d'acylation monocarboxylique ayant quatre ou cinq atomes de carbone, (b) un agent d'acylation monocarboxylique ayant de 7 à 15 atomes de carbone et (c) un agent d'acylation polycarboxylique.

2. Une composition liquide selon la revendication 1, dans laquelle le fluor est le seul halogène dans l'hydrocarbure fluoré (A).

3. Une composition liquide selon l'une des revendications 1 et 2, dans laquelle l'hydrocarbure fluoré (A) est le 1,1,1,2-tétrafluoroéthane.

4. Une composition liquide selon l'une quelconque des revendications précédentes, dans laquelle le composé polyhydroxylé (I) est un triméthylolalcane.

5. Une composition liquide selon l'une quelconque des revendications précédentes, dans laquelle le composé polyhydroxylé (I) est le triméthylpropane.

6. Une composition liquide selon l'une quelconque des revendications précédentes, dans laquelle le composé polyhydroxylé (I) contient une moyenne de cinq groupes hydroxyle ou plus.

7. Une composition liquide selon l'une quelconque des revendications précédentes, dans laquelle le composé polyhydroxylé (I) est le dipenta-érythritol ou le tripenta-érythritol.

8. Une composition liquide selon l'une quelconque des revendications précédentes, dans laquelle l'agent d'acylation monocarboxylique (IIa) est l'acide ou l'anhydride isobutyrique, valérique, 2-méthylbutyrique ou néopentanoïque.

9. Une composition liquide selon l'une quelconque des revendications précédentes, dans laquelle l'agent d'acylation monocarboxylique (IIb) est ramifié.

10. Une composition liquide selon la revendication 9, dans laquelle l'agent d'acylation monocarboxylique (IIb) est un agent d'acylation monocarboxylique ramifié ayant 8 ou 9 atomes de carbone.

11. Une composition liquide selon l'une quelconque des revendications précédentes, dans laquelle l'agent d'acylation polycarboxylique (IIc) est un agent d'acylation dicarboxylique.

12. Une composition liquide selon l'une quelconque des revendications précédentes, dans laquelle l'agent d'acylation polycarboxylique (IIc) est un agent d'acylation choisi parmi des agents d'acylation maléiques, succiniques, adipiques et azélaïques.

13. Une composition liquide selon l'une quelconque des revendications précédentes, comprenant en outre (C) un additif choisi parmi un phosphite d'alcoyle, un ester d'acide alcoyl phosphonique, un hétérocycle contenant de l'azote et leurs mélanges.

14. Une composition liquide selon la revendication 13, dans laquelle (C) est un phosphite d'alcoyle ou un ester d'acide alcoyl phosphonique ayant indépendamment de 1 à 20 atomes de carbone dans chaque groupe alcoyle.

15. Une composition liquide selon la revendication 13, dans laquelle (C) est un phosphite ou un ester d'acide alcoyl phosphonique ayant indépendamment de 3 à 8 atomes de carbone dans chaque groupe alcoyle.

16. Une composition liquide selon la revendication 13, dans laquelle (C) est le phosphite de dibutyle.

17. Une composition liquide selon la revendication 13, dans laquelle (C) est le butylphosphonate de dibutyle.

18. Une composition liquide selon la revendication 13, dans laquelle (C) est un triazole ou un dérivé de triazole.

19. Une composition liquide selon la revendication 13, dans laquelle (C) est le tolyltriazole, le benzotriazole ou un produit de réaction d'un triazole, d'une amine et d'un aldéhyde ou précurseur d'aldéhyde.

20. Une composition liquide selon la revendication 13, dans laquelle (C) est un mélange d'un phosphite d'alcoyle ayant indépendamment de 1 à 20 atomes de carbone dans chaque groupe alcoyle et d'un triazole ou dérivé de triazole.

21. Un procédé de lubrification d'un système de réfrigération comprenant les étapes consistant à introduire dans le système de réfrigération une composition selon l'une quelconque des revendications 1 à 39 et à faire fonctionner le système.

22. Un procédé de préparation d'une composition liquide, comprenant l'opération de mélange de (A) au moins un hydrocarbure fluoré contenant de 1 à 3 atomes de carbone, et d'un lubrifiant comprenant (B) au moins un ester carboxylique de (I) un composé polyhydroxylé et (II) une combinaison de (a) un agent d'acylation monocarboxylique ayant quatre ou cinq atomes de carbone, (b) un agent d'acylation monocarboxylique ayant de 7 à 15 atomes de carbone et (c) un agent d'acylation polycarboxylique.
